# EUROPÄISCHE PATENTSCHRIFT

(11) **EP 1 343 452 B1**
(45) Veröffentlichungstag und Bekanntmachung des Hinweises auf die Patenterteilung: **02.03.2005**
(21) Anmeldenummer: 01271196.6
(22) Anmeldetag: 13.12.2001
(51) Int. Cl.: A61K 6/02, A61K 6/083, C03C 3/095, C03C 3/068

(54) **REAKTIONSTRÄGES DENTALGLAS**
INERT DENTAL GLASS
VERRE DENTAIRE PEU REACTIF

(30) Priorität: 20.12.2000 DE 10063939
(43) Veröffentlichungstag der Anmeldung: 17.09.2003
(73) Patentinhaber: 3M ESPE AG, 82229 Seefeld (DE)
(72) Erfinder: HOESCHELER, Stefan, 82211 Herrsching (DE); MIKULLA, Markus, 82346 Andechs-Frieding (DE); RACKELMANN, Gabriele, 82205 Gilching (DE); BAMBACH, Volker, 79541 Lörrach (DE)
(86) Internationale Anmeldenummer: PCT/EP2001/014721
(87) Internationale Veröffentlichungsnummer: WO 2002/049581

(56) Entgegenhaltungen:
- EP-A- 0 783 872
- WO-A-00/30953
- DE-A- 2 929 121
- DE-A- 3 806 448
- DE-A- 19 914 975
- US-A- 4 772 436
- US-A- 5 849 068

## Beschreibung

Die Erfindung betrifft die Verwendung von reaktionsträgen Gläsern in Dentalmassen, insbesondere Dentalzementen, vorzugsweise Polyelektrolytzementen, die ohne eine Vorbehandlung der Glaspulveroberfläche eingesetzt werden können.

Gläser werden im Dentalbereich insbesondere im Bereich der Füllungsmaterialien, sowie der Befestigungszemente und -composite für Kronen, Brücken und Inlyas verwendet.

Reaktive Gläser, d.h. Gläser, die an einer chemischen Reaktion teilnehmen, kommen in sogenannten Polyelektrolytzementen, insbesondere Glasionomerzementen zum Einsatz.

Solche Polyelektrolytzemente umfassen üblicherweise drei Bestandteile, eine Polysäure, insbesondere eine Carbonsäure-haltige Substanz, vorzugsweise in flüssiger Form, ein Glaspulver und Wasser. Werden die drei Komponenten vereinigt und miteinander gemischt, kommt es zur Reaktion unter Bildung eines über die Zeit aushärtenden festen Körpers (Zementreaktion).

Bei der Herstellung von Gläsern, die insbesondere in Glasionomerzementen verwendet werden, kommen verschiedene Rohstoffe zum Einsatz. Dies sind zum einen Oxide, wie SiO₂, Al₂O₃, CaO, Fluoride, wie CaF₂, SrF₂, Kryolith, Hydroxide, wie Al(OH)₃, Phosphate, wie AlPO₄, P₂O₅ oder Calciumphosphate. Es können aber auch Silikate, wie Mullit oder Karbonate, wie Na₂CO₃, CaCO₃ oder andere mineralische natürliche Rohstoffe verwendet werden. Prinzipiell können alle Rohstoffe auch in kristallwasserhaltiger Form eingesetzt werden.

In Dentalgläsern ist oftmals ein beträchtlicher Anteil des Sauerstoffes durch Fluor ersetzt. Dies wird durch Anfügen des Elementsymbols F für Fluor in der Beschreibung des Glassystems gekennzeichnet.

Demnach können Gläser für Glasionomerzemente üblicherweise einem der folgenden Systeme zugeordnet werden, wobei P₂O₅ und Na₂O in einigen Fällen wenig oder gar nicht vorhanden sind:

SiO₂ - Al₂O₃ - CaO - (P₂O₅) - (Na₂O) - F

SiO₂ - Al₂O₃ - SrO - (P₂O₅) - (Na₂O) - F

SiO₂ - Al₂O₃ - SrO - La₂O₃ - (P₂O₅) - (Na₂O) - F

SiO₂ - Al₂O₃ - CaO - La₂O₃ - (P₂O₅) - (Na₂O) - F

Bei den Gläsern, die in Dentalzementen eingesetzt werden handelt es sich im allgemeinen um Fluoroaluminosilicatgläser. Die Säurelöslichkeit des Glases ist für seine Verwendung als Bestandteil eines Polyelektrolytzements Vorraussetzung. Eine säurelösliche Glasstruktur entsteht dann, wenn Silizium teilweise durch Aluminium ersetzt wird. Das Ersetzen von Silizium durch Aluminium ist jedoch nur bei Anwesenheit von basischen Oxiden möglich, um einen Ladungsausgleich für das 3-wertige Aluminium-Ion auf Plätzen des 4-wertigen Silizium-Ions zu schaffen.

Bei Zugabe der Polysäuren und Wasser wird die Glasstruktur aufgebrochen und insbesondere die Ionen mit netzwerkwandelnden Eigenschaften als sogenannte Vernetzerionen zumindest teilweise freigesetzt.

Die Vernetzung äußert sich in einer über die Zeit zunehmenden Aushärtung des Zementes. Alle mindestens zweiwertigen basischen Ionen, aber auch das Al³⁺ ist zur Ausbildung solcher polymeren Strukturen in der Lage.

Man unterscheidet üblicherweise zwischen der Verarbeitungszeit - dem Zeitpunkt bis zu dem eine Verarbeitung des noch pastösen Zementmasse durch den Zahnarzt möglich ist, und der Aushärtungszeit - dem Zeitpunkt, ab dem eine Nachbearbeitung mit rotierenden Zahnarztinstrumenten möglich wird.

Es hat sich gezeigt, dass herkömmliche Gläser, die als Vernetzerionen beispielsweise Ca²⁺ und Al³⁺ enthalten, unbehandelt zu reaktiv sind und wegen zu hoher Löslichkeit zu schnell mit der Polysäure abbinden, so dass der sich bildende Dentalzement nicht vernünftig verarbeitet werden kann.

Es besteht zwar die Möglichkeit, durch Verringerung des Calciumanteils im Glas den Auflösungsprozess zu verlangsamen; es hat sich jedoch gezeigt, dass sich bei einem zu geringen Anteil basischer Oxide, wie CaO oder SrO, die in Lösung gehen können, die Festigkeitseigenschaften des Zements.auf Grund nicht in ausreichender Menge zur Verfügung stehender lonen verschlechtern. Dies bedeutet, dass für den Zahnarzt eine nur sehr kurze Zeit zur Verfügung steht, die Füllungsmasse zu mischen und zu applizieren. Gleichzeitig hat er den Nachteil in Kauf zu nehmen, sehr lange darauf warten zu müssen, bis er mit der Nachbearbeitung des Zementes beginnen kann. Dies ist konträr zu den Anforderungen, die ein Zahnarzt an einen Dentalzement stellt.

Der Zahnarzt benötigt üblicherweise eine Verarbeitungszeit von 1 bis 4 min und ein Aushärtezeit von 5 bis 8 min. Die Bestimmung der Aushärtezeit erfolgt üblicherweise nach ISO 9917 (First Edition) Teil 7.3. Die Verarbeitungszeit und die Aushärtungszeit lassen sich mit einem Viskosimeter ermitteln, wie es in der EP 0 023 013 A beschrieben ist.

Die EP 0 023 013 A betrifft Calciumaluminiumfluorosilikatglas-Pulver einer bestimmten horngröße und deren Verwendung zur Herstellung von selbsthärtenden Glasionomerzementen.

Um die gewünschten Verarbeitungseigenschaften des Zementes zu erreichen, also genügend Zeit zum Verarbeiten und möglichst wenig Zeit bis zur vollständigen Aushärtung zu haben, ist es üblich, die Glaspulver nach dem Mahlprozess einer Oberflächenbehandlung zu unterziehen, wie es beispielsweise in Clinical Materials 12, 113 - 115 (1993) oder der DE 29 29 121 A (EP 0 230 113 A) beschrieben wird. Hierbei werden die zusammensetzungsbedingt zu schnell reagierenden Gläser durch Verarmung ihrer Oberfläche an reaktiven Ionen in ihrer Reaktionsgeschwindigkeit auf das gewünschte Maß eingestellt.

In der EP 0 023 013 A wird die Verwendung eines Calciumaluminiumfluorosilikatglas-Pulvers für Glasionomerzemente beschrieben, dem weitere Oxide dann zugesetzt werden dürfen, wenn sie die Eigenschaften des Glases nicht beeinträchtigen. Gemäß Beschreibung muss die Oberfläche das Glases deaktiviert werden, um ein brauchbares Glas für einen Dentalzement zu erhalten. Das Deaktivieren stellt einen Vorgang dar, in dem die Reaktionsgeschwindigkeit eines Glaspulvers mit einer Säure durch eine Oberflächenbehandlung verzögert wird und somit die gewünschten Verarbeitungszeiten des Zementes eingestellt werden.

Das Deaktivieren der Oberfläche kann auch durch andere verhältnismäßig aufwendige Oberflächenbehandlungen, wie durch Beschichtung der Oberfläche, beispielsweise mit einem Polymer, erfolgen.

In der EP 0 023 013 A geschieht dies mittels einer chemischen Behandlung der Pulveroberfläche. Es resultiert ein Zement mit günstigen Verarbeitungszeiten, bei gleichzeitig unverändert günstigen mechanischen Werkstoffkenngrößen.

Diese Oberflächenbehandlung der Gläser stellt allerdings einen aufwendigen Verfahrensschritt dar.

Außerdem kann es während der Wasch- oder Temperungsprozessen zu Pulveragglomerationen kommen, die sich nachteilig auf die Zementeigenschaften auswirken.

Aus DE 38 06 448 A ist ein Glas für einen Knochenzement bekannt, das die Elemente Si, Al, Ca, Sr, F, Na und P umfasst und durch einen Zusatz von La₂O₃ röntgensichtbar gemacht werden kann. Es wird hervorgehoben, dass die Menge an Zusätzen die Eigenschaften nicht beeinträchtigen darf.

Das in der DE 38 04 469 A beschriebene Glaspulver ist im wesentlichen frei von Alkaliionen und Erdalkaliionen - ausgenommen Strontium, das in einer Menge von 15 bis 40 Gew.-% eingesetzt werden soll.

In der DE 20 65 824 B2 ist ein Fluoraluminiumsilicatglaspulver für selbsthärtende medizinische Zemente beschrieben

Eine Aufgabe der vorliegenden Erfindung liegt darin, ein Glas für einen Dentälzement, insbesondere ein reaktives Glas für einen Polyelektrolytzement bereitzustellen, dass einfach herzustellen ist.

Eine weitere Aufgabe kann darin gesehen werden, das Glas direkt nach dem Mahlprozess ohne Anwenden von aufwendigen Prozessen, wie Oberflächenbehandlung, Säurewaschen, Beschichten und/oder Tempern direkt zu verwenden. Die Reaktivität und damit die Verarbeitungs- und Aushärtezeit hängen dann nur von Glaszusammensetzung und der Kornverteilung ab und sind auf einfache Weise reproduzierbar herzustellen.

Diese Aufgabe wird durch einen Dentalzement, enthaltend ein Glas, wie es in den Ansprüchen beschrieben ist, und die Verwendung bestimmter Ionen als Vemetzerionen in einem Glas, gelöst.

Gegenstand der Erfindung sind auch Polyelektrolytzemente, die diese Gläser enthalten, sowie ein Verfahren zur Herstellung eines Dentalglasses.

Unter Vernetzung im Sinne der Erfindung ist eine Reaktion zu verstehen, bei der Polysäuren und mindestens zweiwertige Ionen in einer Chelatbildungsreaktion, vorzugsweise einer Säure-Base-artigen Reaktion, miteinander wechselwirken und zur Ausbildung eines polymeren Netzwerkes führen.

In der vorliegenden Erfindung sind unter den genannten Füllungs- und Befestigungsmaterialien im wesentlichen Zemente, und insbesondere Polyelektrolytzemente zu verstehen. Demnach handelt es sich bei dem beschriebenen Glas vorzugsweise um einen reaktiven Bestandteil und keinen klassischen Füllstoff, im Gegensatz zu den im Compositbereich eingesetzten Gläsern, die reine Füllstoffe sind und nicht an einer Reaktion teilnehmen.

Gläser für Zemente enthalten im allgemeinen stark basische Ionen, wie Li⁺, Na⁺, K⁺, Ca²⁺, Sr²⁺, Ba²⁺, Zn²⁺. Es hat sich nun gezeigt, dass durch vollständiges oder teilweises Ersetzen der stark basischen Ionen durch schwach basische Ionen, wie Sc³⁺, Y³⁺, La³⁺ wie Ce^{3+/4+} oder andere 2, 3, 4-wertige Ionen der Lanthanidreihe und/oder Ga²⁺ oder In²⁺, Gläser erhalten werden, die mit Polysäuren wesentlich langsamer abbinden.

Mit solchen Gläsern lassen sich überraschenderweise Dentalzemente herstellen, die im wesentlichen ohne übliche Oberflächenbehandlung der Glaspulver ein Abbindeverhalten aufweisen, welches vom Zahnarzt gewünscht wird. Weiterhin hat sich gezeigt, dass die Abbindezeiten in einem weiten Bereich über die Glaszusammensetzung eingestellt werden können.

Die Erfindung weist dabei folgende Vorteile auf:

Durch Ersetzen der stärk basischen Ionen, wie Ca²⁺, Sr²⁺ durch die schwach basischen Ionen Sc³⁺, Y³⁺, La³⁺ Ce^{4+/3+} und andere 2, 3, 4-wertiger Ionen der Lanthanidreihe in Gläsern, die in Dentalzementen eingesetzt werden, ist eine kontrollierte Abbindereaktion des Dentalzements, insbesondere eines Glasionomerzements erreichbar, ohne, dass das Glas vor seinem Einsatz im Zement, beispielsweise durch Säurewaschung und/oder Tempern oberflächenbehandelt werden muss. Der Vorteil liegt neben der einfacheren Herstellung auch in der besseren Reproduzierbarkeit der Verarbeitungs- und Aushärtezeit. Obwohl diese Zeiten nicht durch eine Oberflächenbehandlung eingestellt werden, erhält man überraschenderweise den gewünschten Abbindeverlauf, nämlich einen verhältnismäßig schnellen Übergang von einem Zustand, in dem der Zement noch verarbeitet werden kann zu einem Zustand, bei dem die Aushärtung beginnt und keine sinnvolle Verarbeitung mehr möglich ist.

Erstaunlicherweise wurde gefunden, dass Dentalmassen bzw. Zemente, in denen diese genannten Gläser eingesetzt werden, die gleichen oder teilweise sogar verbesserte mechanische Eigenschaften aufweisen, als Zemente, in denen Gläsern eingesetzt werden, deren Reaktivität durch Säurewaschung herabgesetzt wurde.

Weiterhin wurde gefunden, dass die erfindungsgemäßen Zemente hydrolytisch beständig gegenüber Wasser sind.

Insbesondere bei Gläsern, die neben Al und Si, nur Y und/oder La enthalten bzw. nur kleinere Mengen an stärker basisch reagierenden lonen, wie Ca ²⁺ oder Sr²⁺, Ba²⁺, Li⁺, Na⁺, K⁺ enthalten, wurden diese Eigenschaften gefunden.

Weiterhin kann ein Teil des Sauerstoffes durch Fluor ersetzt werden, was einerseits die Schmelzbarkeit des Glases und andererseits das Abbindeverhalten des Zements verbessert sowie das Freisetzen von Fluoridionen zur Sekundärkariesprophylaxe ermöglicht.

Die bisher bekannten Systeme der Zemente werden also um folgende Systeme erweitert.

SiO₂ - Al₂O₃ - (SrO) -LnₓO_{y} - P₂O₅ - (Na₂O) - F

SiO₂ - Al₂O₃ - (CaO) -LnₓO_{y} - P₂O₅ - (Na₂O) - F

LnₓO_{y} steht für ein Oxid der Elemente Sc, Y, La bis Lu. x und y können dabei Werte von 1, 2 oder 3 annehmen. Die in Klammern gesetzten Oxide werden nur in geringem Umfang, gegebenenfalls gar nicht eingesetzt, da sie die Reaktion deutlich beschleunigen würden. So beschreibt die DE 20 65 824 A ein Glas des Systems

SiO₂ - Al₂O₃ - La₂O₃ - P₂O₅ - Na₂O - F

mit einem Na₂O Gehalt von ca. 12 Gew.-%.

Versuche haben gezeigt, dass mit diesem Glaspulver nur nach einem mehrstündigen Tempern bei 400 °C die Abbindegeschwindigkeit mit Polysäuren in einen handhabbaren Bereich gebracht werden kann (vgl. Vergleichsbeispiel 4). Dies ist vermutlich auf den hohen Anteil eines stark basischen Oxides, in diesem Fall Na₂O, zurückzuführen. Ein weiterer Nachteil eines hohen Na₂O-Anteils ist die erhöhte Wasserlöslichkeit des resultierenden Zements.

Es wurde außerdem gefunden, dass die beschriebenen Gläser in einem weiten Zusammensetzungsbereich im wesentlichen keine Phasentrennungs- oder Kristallisationseffekte aufweisen.

Es ist zu erwarten, dass die Reproduzierbarkeit der Abbindegeschwindigkeit des die Gläser enthaltenden Zements sich bei klaren Gläsern gegenüber entmischten, also trüben Gläsern verbessert, da deren Phasenbestand nicht von der Abkühlgeschwindigkeit abhängt.

Die Gläser enthalten vorzugsweise neben den üblichen Komponenten SiO₂, Al₂O₃, P₂O₅, und Na₂O im wesentlichen schwach basische und/oder amphoter reagierende Ionen, die während der Zementreaktion als Vernetzerionen wirken.

Bevorzugt sind schwach basische 3- und 4-wertige Ionen und besonders bevorzugt die Ionen Sc³⁺, Y³⁺, La³⁺, Ce^{4+/3+} und alle folgenden 3- und 4-wertigen Ionen der Lanthanidreihe.

Zu den schwach basischen bzw. amphoter reagierenden Ionen gehört nach herrschender Lehrmeinung auch das Al³⁺. Dieses nimmt jedoch bei den Gläsern eine Sonderstellung ein. Aluminium ist in erster Linie für die Säurelöslichkeit der Glasstruktur verantwortlich und wirkt erst in zweiter Linie als Vernetzerion. In den für Dentalzementen geeigneten Gläsern nimmt Aluminium im Gegensatz zu den oben genannten 3- und 4-wertige Ionen, die als Netzwandler fungieren, die Aufgabe eines Netzwerkbildners wahr.

Die verwendeten Gläser weisen üblicherweise eine BET-Oberfläche von 1 bis 15 m²/g, vorzugsweise 2 bis 8 m²/g auf.

Die Gläser haben ferner eine mittlere Korngröße (d₅₀-Wert) von 0,01 bis 20 µm, vorzugsweise 1 bis 5 µm.

Vorzugsweise ist in dem verwendeten Glas ein Anteil von 0 bis 25 Gew.-% des Sauerstoffs durch Fluor ersetzt ist, besonders bevorzugt von 8 bis 18 Gew.-%.

Zur Definition des Begriffes Basizität wird üblicherweise der pK_{B}-Wert herangezogen. Als Grenze zwischen schwach und stark basisch kann ein pK_{B} von 1 angegeben werden. Beispielsweise wird der pK_{B} von Mg(OH)₂ in R. C. Weast: CRC Handbook of Chemistry and Physics mit einem Wert von 1 angegeben, während Ca(OH)₂ ohne Zahlenwertangabe als stark basisch eingestuft wird.

Als schwachbasisch im Sinne der vorliegenden Erfindung gelten Oxide oder Hydroxide, die in wässrigen Lösungen nur zu einem verhältnismäßig geringen Anteil dissoziieren.

Zur Basizität können folgende Aussagen gemacht werden:

Vom Sc über das Y steigt die Basizität zum La an. La ist im Vergleich zu Sr, Ba oder Na und K als schwach basisch einzustufen. Gleichzeitig nimmt die Basizität vom La zum Lu wieder ab, so dass die Basizität des Luthetiums etwa mit der von Yttrium zu vergleichen ist (Lanthanidenkontraktion).

Somit können sämtliche Oxide und Hydroxide der Sc-Reihe als schwach basisch im Sinne der vorliegenden Erfindung bezeichnet werden.

Die Elemente der 1. Hauptgruppe vom Li bis zum Cs und die Elemente der zweiten Hauptgruppe vom Mg bis zum Ba sind im Sinne der vorliegenden Erfindung als nicht schwach basisch reagierend einzustufen.

Neben der Basenstärke spielt vermutlich, wie bereits ausgeführt, auch die höhere Feldstärke dieser Ionen eine gewisse Rolle. Diese bedingt, dass die beschriebenen Ionen in der Glasstruktur stärker verankert sind und somit langsamer herausgelöst werden.

Mit Polysäure im Sinne der vorliegenden Erfindung ist ein Polyelektrolyt gemeint, der ein Polymer mit ionisch dissoziierbaren Gruppen aufweist, die Substituenten der Polymerkette sein können und deren Zahl so groß ist, dass die Polymeren zumindest in ihrer (partiell) dissoziierten Form wenigstens teilweise wasserlöslich sind. Hierfür sind insbesondere Substituenten wie z.B. -COOH, -OH, -PO(OH)₂, -OPO(OH)₂, -SO₂(OH), geeignet. Besonders bevorzugt sind organische Polysäuren (DE 20 61 513 A), wie Polymere und Copolymere der Acrylsäure, Methacrylsäure (EP 0 024 056 A), Itaconsäure, Maleinsäure, Citraconsäure, Vinylphosphonsäure (EP 0 340 016 A; GB 22 91 060 A). Daneben können beim Vorliegen mehrerer Polyelektrolyte auch wasserunlösliche Polyelektrolyte im Polyelektrolytzement vorliegen. Voraussetzung ist lediglich, dass mindestens einer der Polyelektrolyte wenigstens teilweise wasserlöslich ist.

Die Polyelektrolyten sollen mit der Glaskomponente im Sinne einer Chelatbildungsreaktion und/oder einer Säure-Base-Reaktion/Neutralisationsreaktion, reagieren können.

Der Polyelektrolytzement enthält den wenigstens teilweise wasserlöslichen, in den Festzustand überführbaren Polyelektrolyten, vorzugsweise zu einem Anteil von 0,5 bis 30 Gew.-%, besonders bevorzugt 2 bis 25 Gew.-% und ganz besonders bevorzugt 5 bis 20 Gew.-%.

Bei den Polyelektrolytzementen ist der Zusatz von Chelatbildnern zur Einstellung einer geeigneten Abbindung von besonderer Bedeutung (DE 23 19 715 A). Dafür kommen zahlreiche Verbindungen in Frage, vor allem solche, die Chelatbildungen ausbildende Hydroxy- oder Carboxylgruppen oder beide enthalten. Besonders hervorragende Ergebnisse wurden mit Weinsäure oder Citronensäure, insbesondere mit einem Gehalt von 5 Gew.-%, erzielt. Auch der Zusatz in Form eines Metallchelats zeigt den gewünschten Effekt.

In den Polyelektrolytzementen befindet sich 0 bis 10, vorzugsweise 0 bis 5 Gew.-% einer derartigen Verbindung, vorzugsweise Weinsäure.

Ferner kann der Polyelektrolytzement Hilfsstoffe, wie Farbstoffe, Pigmente, Röntgenkontrastmittel, Fließverbesserer, Thixotropiemittel, Polymere Verdickungsmittel oder Stabilisatoren, aufweisen.

Übliche Füllstoffe für Dentalwerkstoffe sind beispielsweise Glas- und Quarzpulver, Kunststoffpulver, pyrogene hochdisperse Kieselsäuren sowie Mischungen dieser Komponenten.

Diese sonstigen Zusätze sind in den erfindungsgemäßen Polyelektrolytzementen üblicherweise zu 0 bis 60 Gew.-% enthalten.

Die genannten Füllstoffe können auch durch eine Oberflächenbehandlung mit Organosilanen bzw. -siloxanen oder durch die Veretherung von Hydroxylgruppen zu Alkoxygruppen hydrophobiert sein.

Prinzipiell sind die erwähnten Glaszusammensetzung auch geeignet, in Monomer-modifzierten Zementen eingesetzt zu werden.

Ein Anteil von 20 bis 70 Gew.-% vorzugsweise von 30 bis 60 Gew.-% an schwach basischen Oxiden im Glas hat sich als günstig erwiesen.

Der erfindungsgemäße Zement enthält gegebenenfalls stark basische Oxide mit einem Anteil im Bereich von 0 bis 25 Gew.-%, vorzugsweise im Bereich von 0 bis 10 Gew.-%.

Der erfindungsgemäße Zement weist vorzugsweise eine Biegefestigkeit im Bereich von mindestens 25 MPa bis 35 MPa auf, besonders bevorzugt von größer 45 MPa, gemessen nach ISO 4049.

Die Verarbeitungszeit des Zements, die mit einem Viskosimeter ermittelt wird, beträgt 1 bis 4 min, besonders bevorzugt 2 bis 3 min. Die Aushärtezeit beträgt 3 bis 10 min, besonders bevorzugt 4 bis 8 min.

Im folgenden werden bevorzugte Zusammensetzungen der Gläser angegeben.

Neben den bereits erwähnten schwach basischen Oxiden der Scandiumreihe können die Gläser noch Oxide der 4. Und 5. Nebengruppe enthalten. Auch kann das Aluminiumoxid teilweise oder vollständig durch Bor- oder Galliumoxid ersetzt werden. Die Schmelzbedingungen können durch Zugabe von Oxiden der 1. Hauptgruppe, Phosphat und/oder basischen Oxiden der 2. Hauptgruppe oder ZnO positiv beeinflusst werden.

Die in der Tabelle durch "+" von einander abgegrenzten Oxide können erfindungsgemäß auch nur einzeln vorliegen. Entscheidend ist der jeweilige Gewichtsanteil der Gruppe am Glas.

| Oxid | Anteil |
|---|---|
| Y₂O₃ + La₂O₃+ andere Lanthanidoxide oder Sc₂O₃ | 30 bis 80 Gew.-%, vorzugsweise 35 bis 60 Gew.-% 20 bis 50 Gew.% |
| B₂O₃ + Al₂O₃+ Ga₂O₃ | 5 bis 50 Gew.-%, vorzugsweise 10 bis 40 Gew.-%, besonders bevorzugt 15 bis 35 |
| SiO₂ + GeO₂ + SnO | 10 bis 50 Gew.-%, vorzugsweise 15 bis 50 Gew.-% |
| P₂O₅ | 0 bis 15 Gew.-%, vorzugsweise 0 bis 10 Gew.-%, besonders bevorzugt 0 bis 2 Gew.-% |
| MgO + CaO + SrO + ZnO + BaO | 0 bis 10 Gew.-%, vorzugsweise 0 bis 8 Gew.-%, besonders bevorzugt 0 bis 5 Gew.-% |
| Li₂O + Na₂O + K₂O + Rb₂O + Cs₂O | 0 bis 5 Gew.-%, vorzugsweise 0 bis 3 Gew.-%, besonders bevorzugt 0 bis 2 Gew.-% |
| TiO₂ + ZrO₂ + HfO₂ | 0 bis 10 Gew.-%, vorzugsweise 0 bis 4 Gew.-% |
| V₂O₅ + Nb₂O₅ + Ta₂O₅ | 0 bis 10 Gew.-%, vorzugsweise 0 bis 4 Gew.-%, |

An Stelle des Bestandteils "Y₂O₃+ La₂O₃+ andere Lanthanidoxide" kann auch Sc₂O₃ in einer Menge von 20 bis 50 Gew.-%, vorzugsweise von 20 bis 30 Gew.-% enthalten sein kann. Umfasst sind auch Glaszusammensetzungen, in denen Sc₂O₃ neben obigem Bestandteil in vergleichsweise kleiner Menge zusätzlich enthalten ist.

Die Erfindung wird nachfolgend anhand einiger Beispiele näher beschrieben:

Keines der Gläser der Beispiele wurde vor seiner Umsetzung mit einer Polysäure mit einer anorganischen Säure behandelt, die zu einer Verarmung der Oberfläche der Gläser an reaktiven Ionen führt (Säurewaschung).

### Glasherstellung:

Gläser mit folgenden oxidischen Zusammensetzungen (in Gew.-%) wurden bei Temperaturen im Bereich von 1300 bis 1600 °C über einen Zeitraum von 30 min bis 5 h geschmolzen. Mit Ausnahme von Vergleichsbeispiel 4 betrug der Fluorgehalt 12 bis 14 Gew.-% in der Einwaage.

In Vergleichsbeispiel 4 wurde ein Glas gemäß DE 20 65 824 A1 unter Verwendung folgender Zusammmensetzung geschmolzen:

9,5 g SiO₂, 10,0 g Al₂O₃, 7,6 g Na₃AlF₆, 9,4 g LaF₃, 7,3 g AlPO₄. In der Tabelle ist hierzu die oxidische Zusammensetzung angegeben.

| | **1** | **2** | **3** | **4** | **5** | **6** | **7** | **8** | **9** | **V1** | **V2** | **V3** | **V4** | **V5** | **V6** | **V7** |
|---|---|---|---|---|---|---|---|---|---|---|---|---|---|---|---|---|
| SiO₂ | 20 | 19 | 20 | 17 | 32 | 27 | 13 | 21 | 19 | 20 | 18 | 46 | 23 | 17 | 26 | 29 |
| Sc₂O₃ | | | | | | | | | | | | | | | | |
| Y₂O₃ | 36 | | 58 | | 20 | 41 | 50 | 46 | 49 | | | | | 48 | | 30 |
| La₂O₃ | | 48 | | 63 | 20 | | | | | 34 | | | 22,5 | | 23 | |
| CaO | | 7 | | | | | | | | 16 | 47 | | | 3 | 16 | |
| SrO | | | | | | | | | | | | 17 | | | | |
| Al₂O₃ | 44 | 26 | 22 | 20 | 28 | 32 | 37 | 28 | 26 | 29 | 34 | 35 | 36 | 26 | 30 | 27 |
| P₂O₅ | | | | | | | | | 6 | | | | 10 | | 5 | |
| ZrO2 | | | | | | | | 5 | | | | | | | | |
| Na₂O | | 1 | | | | | | | | 1 | 1 | 2 | 8,5 | 6 | | 1 |
| Li2O | | | | | | | | | | | | | | | | 13 |

### Mahlung

Von dem erhaltenen Glasgranulat wurden 60 bis 80 g in einer Achatschwingmühle (Fa. Siebtechnik, Mahlgefäß, 100 ml, 910 U/min) mit einer Dauer von 40 bis 50 min trocken gemahlen. Die erhaltenen Glaspulver wiesen eine mittlere Korngröße im Bereich von 3 bis 6 µm bei einer spezifischen Oberfläche von 1,8 bis 2,5 m²/g auf.

### Beispiel 6:

Glasbeispiel 6 wurde zusätzlich mit einer Rührwerkskugelmühle nass gemahlen. Es wurde ein Aluminiumoxidgefäß (500 ml) mit 50 g Glaspulver 200 ml H₂O und 100 g Zirkonoxidkugeln (D=0,8 mm) gefüllt und 6 h mit einer gelochten Zirkonoxidscheibe gemahlen. Es ergab sich eine mittlere Korngröße von 1,5 µm und eine spezifische Oberfläche von 10,5 m².

### Zemente

Die Zemente wurden unter Mischen der erhaltenen Glaspulver mit Polysäuren hergestellt. Dabei kam eine ca. 45 %ige Polyacrylsäure (Molekülgewicht 20000 bis 30000) eine ca. 45 %ige Polyacrylmaleinsäure (Molekülgewicht ca. 40000 bis 60000) und eine ca. 55 %ige Polyvinylphosphonsäure (Molekülgewicht ca. 20 000) zum Einsatz.

Die Abbindung wurde zum einen in Anlehnung an ISO 9917, zum anderen mit dem in der EP 0 023 013 A1 beschriebenen Viskosimeter bestimmt. In allen Fällen wurde eine Temperierung der Proben auf 28 °C durch den Versuchsaufbau gewährleistet. Biegefestigkeiten wurden im Dreipunktbiegeversuch an 2x2x25 mm Zementproben nach ISO 4049 bestimmt.

### Ergebnisse:

### Zement 1:

Glas 1 wurde sowohl mit einer Polyacryl- als auch einer Polyacrylmaleinsäure im P:F von 3:1 gemischt.

| | Polyacrylsäure | Polyacrylmaleinsäure |
|---|---|---|
| Viskosimeter (Verarbeitungszeit) | 3:30 / | 3:50 |
| Viskosimeter (Aushärtezeit) | 9:10 | 9:00 |
| ISO 9117 | 8:30 | 7:30 |
| Biegefestigkeit [MPa] | 39,4 | 31,8 |

### Zement 6:

Glas 6 wurde mit Polyacrylsäure (45 %ig) im P:F von 2,0:1 umgesetzt.

| | Trockenmahlung | Rührwerkskugelmahlung |
|---|---|---|
| Viskosimeter (Verarbeitungszeit) | 5:00 | 2:10 |
| Viskosimeter (Aushärtezeit) | 12:00 | 7:45 |
| ISO 9117 | 10:30 | 4:00 |
| Biegefestigkeit | 27,9 MPa | 41,5 MPa |

### Glas V6:

Das Glas wurde einmal unbehandelt und einmal nach 6-stündiger Temperung bei 400 °C in einem Umluftofen (Fa. Heraeus) mit Polyacrylsäure umgesetzt.

| | unbehandelt | getempert |
|---|---|---|
| Viskosimeter (Verarbeitungszeit) | nicht bestimmbar | 1:50 |
| Viskosimeter (Aushärtezeit) | | 5:20 |
| ISO 9117 | nicht bestimmbar | 5:00 |
| Biegefestigkeit | nicht bestimmbar | 37,4 MPa |

| Weitere Zementbeispiele: | | | | | | | | | | | | | |
|---|---|---|---|---|---|---|---|---|---|---|---|---|---|
| **Glas** | **2** | **3** | **4** | **5** | **7** | **8** | **9** | **V1** | **V2** | **V3** | **V4** | **V5** | **V7** |
| P:F | 3:1 | 3:1 | 3:1 | 3:1 | 3,5:1 | 4:1 | 4:1 | 3:1 | 3:1 | 3:1 | 3:1 | 4:1 | 4:1 |
| Viskosimeter (Verarbeit.-zeit) | 1:30 | 2:50 | 2:40 | 3:20 | 4:00 | 3:50 | 2:50 | 0:45 | <1:0 | <1:0 | <1:0 | <1:0 | <1:0 |
| Viskosimeter (Aushärtezeit) | 3:50 | 8:20 | 5:20 | 7:40 | 9:10 | 8:50 | 6:45 | 1:30 | <1:0 | <1:0 | <1:0 | <1:0 | <1:0 |
| ISO 9917 | 4:00 | 7:15 | | 5:00 | 8:30 | 7:40 | 6:00 | 1:00 | <1:0 | <1:0 | <1:0 | <1:0 | <1:0 |
| Dreipunktbiegefestigkeit | 37,4 | 41,6 | | 34,8 | 35,6 | 32,9 | 43,9 | - | - | - | - | - | - |

Die bei den in den Beispielen 1 bis 9 erhaltenen Zementen ermittelten Abbindezeiten liegen alle im bevorzugten Bereich, mit Ausnahme des Glases 6, das nur trocken gemahlen wurde.

Der Zement gemäß Vergleichsbeispiel 1 bindet vermutlich bedingt durch den hohen Ca-Anteil zu rasch ab. Bei den Zementen gemäß den Vergleichsbeispielen 2 und 3 waren Messung bedingt durch zu schnelles Abbinden nicht mehr möglich.

Bei den Messungen mit dem Viskosimeter entspricht die erste Zeitangabe der Verarbeitungszeit, die zweite Zeitangabe der Aushärtungszeit. Die Zeitangaben beziehen sich auf Minuten. Das P:F-Verhältnis ist als Gewichtsverhältnis angegeben.

Die erfindungsgemäßen Zemente werden üblicherweise in Behältnissen abgepackt in den Handel gebracht. Hierbei ist darauf zu achten, dass die einzelnen Komponenten des Zementes so vorliegen, dass keine ungewollte Reaktion vor dem bestimmungsgemäßen Gebrauch erfolgt. Die Behältnisse weisen üblicherweise mindestens zwei von einander getrennte Kammern auf. Geeignete Behältnisse sind beispielsweise in der WO 00/30953 A oder der EP 0 783 872 A beschrieben.

Geeignete Behältnisse sind Mischkapseln und verschließbare dosenförmige Hohlkörper, wie Schraubdeckelgläser. Je nach Anwendungsbereich können die Zemente auch in Kapseln abgepackt werden.

## Patentansprüche

1. Dentalzement, umfassend ein Dentalglas **gekennzeichnet durch** eine Zusammensetzung:
| Oxid | Anteil |
|---|---|
| Y₂O₃ und/oder La₂O₃ und/oder andere Lanthanidoxide oder Sc₂O₃ | 30 bis 80 Gew.-% 20 bis 50 Gew.-% |
| B₂O₃ und/oder Al₂O₃ und/oder Ga₂O₃ | 5 bis 50 Gew.-% |
| SiO₂ und/oder GeO₂ und/oder SnO | 10 bis 50 Gew.-% |
| P₂O₅ | 0 bis 18 Gew.-% |
| MgO und/oder CaO und/oder SrO und/oder ZnO und/oder BaO und/oder Li₂Ound/oder Na₂O und/oder K₂O und/oder Rb₂O und/oder Cs₂O | 0 bis 10 Gew.-% |
| TiO₂ und/oder ZrO₂ und/oder HfO₂ | 0 bis 10 Gew.-% |
| V₂O₅ und/oder Nb₂O₅ und/oder Ta₂O₅ | 0 bis 10 Gew.-% |

2. Dentalzement nach Anspruch 1, umfassend ein Dentalglas **gekennzeichnet durch** eine Zusammensetzung:
| Oxid | Anteil |
|---|---|
| Sc₂O₃ und/oder Y₂O₃ und/oder La₂O₃ und/oder andere Lanthanidoxide oder Sc₂O₃ | 30 bis 60 Gew.-% 20 bis 50 Gew.-% |
| Al₂O₃ | 15 bis 40 Gew.-% |
| SiO₂ | 15 bis 50 Gew.-% |
| P₂O₅ | 0 bis 2 |
| MgO und/oder CaO und/oder SrO und/oder ZnO und/oder BaO | 0 bis 8 Gew.-% |
| Li₂Ound/oder Na₂Ound/oderK₂Ound/oder Rb₂Ound/oderCs₂O | 0 bis 2 Gew.-% |
| TiO₂und/oderZrO₂und/oder HfO₂ | 0 bis 4 Gew.-% |
| V₂O₅ und/oder Nb₂O₅ und/oder Ta₂O₅ | 0 bis 4 Gew.-% |
wobei an Stelle des Bestandteils Y₂O₃und/oder La₂O₃und/oder andere Lanthanidoxide auch Sc₂O₃ in einer Menge von 20 bis 50 Gew.-% enthalten sein kann.

3. Dentalzement nach einem der vorstehenden Ansprüche, wobei das Glas im wesentlichen ein Dreistoffsystem ist.

4. Dentalzement nach einem der vorstehenden Ansprüche, wobei ein Anteil von 0 bis 25 Gew.-% des Sauerstoffs im Dentalglas durch Fluor ersetzt ist.

5. Dentalzement nach einem der vorstehenden Ansprüche, wobei das Dentalglas in Pulverform mit einer spezifischen BET-Oberfläche von 1 bis 15 m²/g vorliegt.

6. Dentalzement nach einem der vorstehenden Ansprüche, wobei das Dentalglas eine mittlere Korngröße im Bereich von 0,01 bis 10 µm aufweist.

7. Dentalzement nach einem der vorstehenden Ansprüche, wobei die Oberfläche des Dentalglases zur Einstellung der Abbindezeit nicht oberflächenbehandelt wurde, beispielsweise durch Waschen mit Säure, Oberflächenbeschichten und/oder Tempern.

8. Dentalzement, umfassend A) mineralischen Feststoff in einer Menge von 50 bis 90 Gew.-%, B) Wasser in einer Menge von 5 bis 50 Gew.-% und C) Polysäure in einer Menge von 5 bis 50 Gew.-%, wobei der mineralische Feststoff ein Dentalglas, wie es in einem der Ansprüche 1 bis 8 beschrieben ist und ggf. einen Füllstoff umfasst.

9. Dentalzement nach Anspruch 8, wobei in Komponente A) der Füllstoff in einer Menge von 0 bis 90 Gew.-% vorliegt.

10. Dentalzement nach Anspruch 9, wobei der Füllstoff gewählt ist aus Quarz, Gläser, Aluminiumoxid, mineralische Pulver, wie Feldspäte oder Kaolin und/oder Kunststoffpulver.

11. Dentalzement nach einem der Ansprüche 8 bis 10 mit einer Biegefestigkeit von mindestens 25 MPa, gemessen nach ISO 4049.

12. Behältnis mit mindestens zwei Kammern, enthaltend einen Dentalzement nach einem der Ansprüche 8 bis 11, wobei die fließfähigen Bestandteile von den festen Bestandteilen getrennt sind.

13. Behältnis nach Anspruch 12 in Form einer Mischkapsel.

14. Verfahren zur Herstellung eines Dentalglases wie es in einem der vorstehenden Ansprüche 1 bis 7 beschrieben ist, umfassend die Schritte a) Bereitstellen der oxidischen Substanzen, b) Mischen der oxidischen Substanzen, c) Schmelzen der Mischung aus Schritt b), d) Abschrecken der Schmelze zu einem Festkörper, e) Mahlen des Festkörpers aus Schritt d) zu einem Glaspulver, wobei das Glaspulver aus Schritt d) vor dem Einsatz in einem Dentalzement nicht mit Säure behandelt wird.

15. Verwendung eines Dentalglases wie es in einem der vorstehenden Ansprüche 1 bis 7 beschrieben ist zur Herstellung eines Zements, wobei die Oberfläche des Glases nicht oberflächenbehandelt wurde.

16. Verwendung nach Anspruch 15, wobei es sich bei dem Zement um einen Polyelektrolytzement handelt.

17. Verwendung von Ionen schwach basisch reagierender Oxide in Gläsern, die eine Zementreaktion mit einer Polysäure eingehen können, zum Vemetzen der Polysäure, wobei die Oxide in einer Menge von mindestens 20 Gew.-% eingesetzt werden, und wobei die Ionen der Oxide gewählt sind aus Sc³⁺, Y³⁺, La³⁺, Ce⁴⁺ und allen folgenden drei- und vierwertigen Lanthaniden sowie Ga²⁺ und/oder In²⁺.

18. Verwendung nach Anspruch 17, wobei die Oxide einen pK_{B}-Wert von > 1 aufweisen.

## Claims

1. Dental cement comprising a dental glass **characterized by** the following composition:
| Oxide | Proportion |
|---|---|
| Y₂O₃ and/or La₂O₃ and/or other lanthanide oxides Sc₂O₃ | 30 to 80% by weight 20 to 50% by weight |
| B₂O₃ and/or Al₂O₃ and/or Ga₂O₃ | 5 to 50% by weight |
| SiO₂ and/or GeO₂ and/or SnO | 10 to 50% by weight |
| P₂O₅ | 0 to 18% by weight |
| MgO and/or CaO and/or SrO and/or ZnO and/or BaO and/or Li₂O and/or Na₂O and/or K₂O and/or Rb₂O and/or Cs₂O | 0 to 10% by weight |
| TiO₂ and/or ZrO₂ and/or HfO₂ | 0 to 10% by weight |
| V₂O₅ and/or Nb₂O₅ and/or Ta₂O₅ | 0 to 10% by weight |

2. Dental cement according to Claim 1, comprising a dental glass **characterized by** the following composition:
| Oxide | Proportion |
|---|---|
| Sc₂O₃ and/or Y₂O₃ and/or La₂O₃ and/or other lanthanide oxides Sc₂O₃ | 30 to 60% by weight 20 to 50% by weight |
| Al₂O₃ | 15 to 40% by weight |
| SiO₂ | 15 to 50% by weight |
| P₂O₅ | 0 to 2 |
| MgO and/or CaO and/or SrO and/or ZnO and/or BaO | 0 to 8% by weight |
| Li₂O and/or Na₂O and/or K₂O and/or Rb₂O and/or Cs₂O | 0 to 2% by weight |
| TiO₂ and/or ZrO₂ and/or HfO₂ | 0 to 4% by weight |
| V₂O₅ and/or Nb₂O₅ and/or Ta₂O₅ | 0 to 4% by weight |
in which instead of the Y₂O₃ and/or La₂O₃ and/or other lanthanide oxides it is also possible for Sc₂O₃ to be present in an amount of 20 to 50%.

3. Dental cement according to one of the preceding claims, in which the glass is essentially a three-component system.

4. Dental cement according to one of the preceding claims, in which from 0 to 25% by weight of the oxygen in the dental glass is replaced by fluorine.

5. Dental cement according to one of the preceding claims, in which the dental glass is present in powder form with a specific BET surface area of 1 to 15 m²/g.

6. Dental cement according to one of the preceding claims, in which the dental glass has a mean grain size in the range from 0.01 to 10 µm.

7. Dental cement according to one of the preceding claims, in which the surface of the dental glass has not been surface-treated, for example by washing with acid, surface coating and/or conditioning, in order to adjust the setting time.

8. Dental cement, comprising A) mineral solid in an amount of from 50 to 90% by weight, B) water in an amount of from 5 to 50% by weight, and C) polyacid in an amount of from 5 to 50% by weight, the mineral solid comprising a dental glass as described in one of claims 1 to 8 and optionally a filler.

9. Dental cement according to Claim 8, in which in component A) the filler is present in an amount of from 0 to 90% by weight.

10. Dental cement according to Claim 9, in which the filler is selected from quartz, glasses, aluminium oxide, mineral powders, such as feldspars or kaolin, and/or plastic powder.

11. Dental cement according to one of Claims 8 to 10 with a flexural strength of at least 25 Mpa, measured according to ISO 4049.

12. Vessel having at least two chambers, containing a dental cement according to one of Claims 8 to 11, in which the free-flowing constituents are separated from the solid constituents.

13. Vessel according to Claim 12 in the form of a mixing capsule.

14. Process for producing a dental glass as described in one of the preceding Claims 1 to 7, comprising the steps a) providing the oxidic substances, b) mixing the oxidic substances, c) melting the mixture from step b), d) quenching the molten material to form a solid, e) milling the solid from step d) to form a glass powder, the glass powder from step d) not being treated with acid before it is used in a dental cement.

15. Use of a dental glass as described in one of the preceding Claims 1 to 7 for production of a cement, without the surface of the glass having being surface-treated.

16. Use according to Claim 15, in which the cement is a polyelectrolyte cement.

17. Use of ions of weakly basic-reacting oxides in glasses, which can enter into a cement reaction with a polyacid, to crosslink the polyacid, the oxides being used in an amount of at least 20% by weight, and the ions of the oxides being selected from Sc³⁺, Y³⁺, La³⁺, Ce⁴⁺ and all the following trivalent and tetravalent lanthanides and Ga²⁺ and/or In²⁺.

18. Use according to Claim 17, in which the oxides have a pK_{B} value of > 1.

## Revendications

1. Ciment dentaire, comprenant un verre dentaire **caractérisé par** la composition :
| Oxyde | Proportion |
|---|---|
| Y₂O₃ et/ou La₂O₃ et/ou autres oxydes de lanthanides ou Sc₂O₃ | 30 à 80 % en poids 20 à 50 % en poids |
| B₂O₃ et/ou Al₂O₃ et/ou Ga₂O₃ | 5 à 50 % en poids |
| SiO₂ et/ou GeO₂ et/ou SnO | 10 à 50 % en poids |
| P₂O₅ | 0 à 18 % en poids |
| MgO et/ou CaO et/ou SrO et/ou ZnO et/ou BaO et/ou Li₂O et/ou Na₂O et/ou K₂O et/ou Rb₂O et/ou Cs₂O | 0 à 10 % en poids |
| TiO₂ et/ou ZrO₂ et/ou HfO₂ | 0 à 10 % en poids |
| V₂O₅ et/ou Nb₂O₅ et/ou Ta₂O₅ | 0 à 10 % en poids |

2. Ciment dentaire selon la revendication 1, comprenant un verre dentaire, **caractérisé par** la composition :
| Oxyde | Proportion |
|---|---|
| Sc₂O₃ et/ou Y₂O₃ et/ou La₂O₃ et/ou autres oxydes de lanthanides ou Sc₂O₃ | 30 à 60 % en poids 20 à 50 % en poids |
| Al₂O₃ | 15 à 40 % en poids |
| SiO₂ | 15 à 50 % en poids |
| P₂O₅ | 0 à 2 % en poids |
| MgO et/ou CaO et/ou SrO et/ou ZnO et/ou BaO | 0 à 8 % en poids |
| Li₂O et/ou Na₂O et/ou K₂O et/ou Rb₂O et/ou Cs₂O | 0 à 2 % en poids |
| TiO₂ et/ou ZrO₂ et/ou HfO₂ | 0 à 4 % en poids |
| V₂O₅ et/ou Nb₂O₅ et/ou Ta₂O₅ | 0 à 4 % en poids |
dans lequel, à la place du constituant Y₂O₃ et/ou La₂O₃ et/ou autres oxydes de lanthanides peut être inclus aussi Sc₂O₃ en une quantité de 20 à 50 % en poids.

3. Ciment dentaire selon l'une quelconque des revendications précédentes, dans lequel le verre est essentiellement un système à trois substances.

4. Ciment dentaire selon l'une quelconque des revendications précédentes, dans lequel une proportion de 0 à 25 % en poids de l'oxygène dans le verre dentaire peut être remplacée par du fluor.

5. Ciment dentaire selon l'une quelconque des revendications précédentes, dans lequel le verre dentaire se présente sous forme de poudre avec une surface spécifique BET de 1 à 15 m²/g.

6. Ciment dentaire selon l'une quelconque des revendications précédentes, dans lequel le verre dentaire présente une taille moyenne de grains dans la plage de 0,01 à 10 µm.

7. Ciment dentaire selon l'une des revendications précédentes, dans lequel la surface du verre dentaire n'a pas été traitée en surface pour l'ajustement du temps de prise, par exemple par lavage avec un acide, revêtement de surface et/ou recuit.

8. Ciment dentaire comprenant A) une substance solide minérale en une quantité de 50 à 90 % en poids, B) de l'eau en une quantité de 5 à 50 % en poids et C) un polyacide en une quantité de 5 à 50 % en poids, la substance solide minérale étant un verre dentaire, comme cela est décrit dans l'une quelconque des revendications 1 à 8 et éventuellement comprend une charge.

9. Ciment dentaire selon la revendication 8, dans lequel dans le composant A) la charge se présente en une quantité de 0 à 90 % en poids.

10. Ciment dentaire selon la revendication 9, dans lequel la charge est choisie parmi le quartz, les verres, l'oxyde d'aluminium, les poudres minérales, comme les feldspaths ou le kaolin et/ou les poudres de matières synthétiques.

11. Ciment dentaire selon l'une quelconque des revendications 8 à 10, avec une résistance à la flexion d'au moins 25 MPa, mesurée selon la norme ISO 4049.

12. Récipient avec au moins deux chambres, contenant un ciment dentaire selon l'une quelconque des revendications 8 à 11, dans lequel les constituants pouvant s'écouler sont séparés des constituants solides.

13. Récipient selon la revendication 12 sous la forme d'une capsule de mélange.

14. Procédé pour la préparation d'un verre dentaire comme cela est décrit dans l'une quelconque des revendications 1 à 7 précédentes, comprenant les étapes consistant à a) préparer les substances oxydées, b) mélanger les substances oxydées, c) fondre le mélange de l'étape b), d) tremper la fonte en un solide, e) broyer le solide de l'étape d) en une poudre de verre, la poudre de verre de l'étape d) n'étant pas traitée à l'acide avant l'utilisation dans un ciment dentaire.

15. Utilisation d'un verre dentaire comme décrit dans l'une quelconque des revendications 1 à 7 précédentes pour la préparation d'un ciment, la surface du verre n'ayant pas été traitée en surface.

16. Utilisation selon la revendication 15, dans laquelle il s'agit pour le ciment d'un ciment polyélectrolytique.

17. Utilisation d'ions d'oxydes réagissant faiblement basiquement dans des verres, qui peuvent entrer dans une réaction de ciment avec un polyacide, pour la réticulation du polyacide, les oxydes étant mis en oeuvre en une quantité d'au moins 20 % en poids, et les ions des oxydes étant choisis parmi Sc³⁺, Y³⁺, La³⁺, Ce⁴⁺ et tous les lanthanides suivants tri- et quadrivalents ainsi que Ga²⁺ et/ou In²⁺.

18. Utilisation selon la revendication 17, dans laquelle les oxydes présentent une valeur de pK_{B} > 1.
